# EUROPEAN PATENT SPECIFICATION

(11) **EP 4 311 856 B1**
(45) Date of publication and mention of the grant of the patent: **22.10.2025**
(21) Application number: 23187343.1
(22) Date of filing: 24.07.2023
(51) Int. Cl.: C12N 1/20, A01N 63/20

(54) **STRAINS OF THE GENUS PANTOEA SPP. AND THE USE OF STRAINS OF THE GENUS PANTOEA SPP. IN PLANT PROTECTION**
STÄMME DER GATTUNG PANTOEA SPP. VERWENDUNG VON STÄMMEN DER GATTUNG PANTOEA SPP. IN-PLANT-SCHUTZ
SOUCHES DU GENRE PANTOEA SPP. UTILISATION DE SOUCHES DU GENRE PANTOEA SPP. PROTECTION D'INSTALLATION

(30) Priority: 25.07.2022 PL 44183422
(43) Date of publication of application: 31.01.2024
(60) Divisional application: 24222316.2
(73) Proprietor: Instytut Ogrodnictwa - Panstwowy Instytut Badawczy, 96-100 Skierniewice (PL); INTERMAG SP. Z O.O, 32-300 Olkusz (PL)
(72) Inventor: Kardasz, Hubert, 32-300 Olkusz (PL); Goralska, Katarzyna, 31-226 Krakow (PL); Oleszczak, Marcin, 25-640 Kielce (PL); Ambroziak, Krzysztof, 31-345 Krakow (PL); Mikicinski, Artur, 96-100 Skierniewice (PL); Pulawska, Joanna, 96-100 Skierniewice (PL); Jarecka - Boncela, Anna, 96-100 Skierniewice (PL); Ptaszek, Magdalena, 94-002 Lodz (PL); Wlodarek, Agnieszka, 96-130 Gluchow (PL); Poniatowska, Anna, 96-100 Skierniewice (PL); Michalecka, Monika, 96-100 Skierniewice (PL); Kaluzna, Monika, 96-100 Skierniewice (PL); Glos, Hubert, 96-100 Skierniewice (PL)
(74) Representative: Belz, Anna

(56) References cited:
- WO-A1-2021/028911
- KR-A- 20080 007 038
- WALTERSON ALYSSA M ET AL: "Pantoea: insights into a highly versatile and diverse genus within the Enterobacteriaceae", FEMS MICROBIOLOGY REVIEWS, vol. 027, no. 39, 1 January 2015 (2015-01-01), pages 968 - 984, XP055797339, Retrieved from the Internet <URL:https://watermark.silverchair.com/fuv027.pdf?token=AQECAHi208BE49Ooan9kkhW_Ercy7Dm3ZL_9Cf3qfKAc485ysgAAAqEwggKdBgkqhkiG9w0BBwagggKOMIICigIBADCCAoMGCSqGSIb3DQEHATAeBglghkgBZQMEAS4wEQQME-iwqthDWpraND4KAgEQgIICVB0mh9tf26GpjvlykSgB7oocFtmpF9cku6Ta8wGKY3opP6uTVX_RY9xAPKQBZ-sGDnm-xmkryJaRX4GbV0R7qmH9gLSnC> DOI: 10.1093/femsre/fuv027
- BRADY CARRIE L. ET AL: "Pantoea allii sp. nov., isolated from onion plants and seed", INTERNATIONAL JOURNAL OF SYSTEMATIC AND EVOLUTIONARY MICROBIOLOGY, vol. 61, no. 4, 1 April 2011 (2011-04-01), GB, pages 932 - 937, XP093075673, ISSN: 1466-5026, DOI: 10.1099/ijs.0.022921-0

## Description

The subject of the invention is Strain T16/8 of *Pantoea agglomerans* and the application of strain *Pantoea agglomerans* T16/8 in plant protection of vegetable, fruit, agricultural, and ornamental plants against fungal diseases such as, for example, white mold, gray mold, alternariosis, anthracnose, brown rot, bull's eye rot, powdery mildew, and blue mold, as well as bacterial diseases such as, for example, fire blight or soft rot, affecting cultivated plants during the vegetation period and throughout the storage of harvest.

One of the main goals of agriculture in highly developed countries is to reduce the use of chemical pesticides by implementing eco-friendly production systems. The widespread adoption and implementation of these systems allow for restoring environmental balance and utilizing its natural resistance against pests and other harmful factors that limit the efficiency of crop production. In society, there is a growing expectation for the development of the methods alternative to chemical approaches. One of the solutions to reduce the chemical impact of agriculture is the application of bioproducts - plant protection agents based on microorganisms that enable plant defense against diseases while also ensuring the production of consumer-safe fruits and vegetables with reduced residues of chemical pesticides.

One of the most important and commonly occurring fungal diseases in cultivated plants is white rot, caused by the fungus *Sclerotinia sclerotiorum.* This fungus is a polyphagous pathogen, affecting most vegetable species, and it is also found in the cultivation of crops like rapeseed, sunflower, and soybean. The greatest damage is inflicted by the pathogen on plants that produce storage organs such as roots, bulbs, or tubers. In the case of cabbage vegetables, *S. sclerotiorum* can cause post-harvest rot of the heads during storage. White mold also poses a significant threat to seed plantations, not only of root vegetables but also cabbage vegetables and agricultural crops. A significant challenge for growers is the effective eradication of the pathogen from cultivation, as the disease-causing agent produces numerous sclerotia, which have a lifespan of several years. As a result, they can persistently contaminate the soil or the storage facilities where harvested vegetables are kept.

Gray mold caused by the fungus *Botrytis cinerea* attacks numerous plant species, and it can occur throughout the entire vegetation period, affecting all of their above-ground parts. The disease-causing agent affects agricultural, fruit-bearing, and ornamental plants. Berry plants, in particular, are highly susceptible to infections. The pathogen can cause damage even during the germination phase of plants, leading to damping-off and the death of young seedlings. Gray mold also affects vegetables during storage. The fungus requires high relative humidity in the air and heavily moistened leaves for infection and development. The disease's progression is facilitated by low light levels, plant weakening due to other diseases, as well as calcium and potassium deficiency in the soil.

Alternariosis diseases constitute a group of leaf, stem, and to a lesser extent, fruit diseases caused by fungi belonging to the *Alternaria* spp. genus. These microorganisms are widely spread in major cultivation regions of onions, cabbage vegetables, leeks, carrots, potatoes, and tomatoes. Infections caused by *Alternaria* spp. spores can lead to significant losses in the quality and quantity of harvested crops and contribute to their poorer storage. A characteristic feature of diseases caused by fungi of the *Alternaria* spp. is the presence of concentric, slightly sunken, small spots ranging in color from green to dark brown. These spots are centrally arranged on the affected plant organs. Over time, the spots transform into dark brown angular forms with a yellow border.

Anthracnose is a fruit disease caused by the fungus *Glomerella acutata,* primarily known in its conidial stage as *Colletotrichum acutatum,* affecting fruits at various stages of their growth. Symptoms can be observed both in orchards and during storage. The fungus overwinters in the form of mycelium in cankers on the bark and occasionally on mummified fruits. During the growth of apples and pears in conditions of high humidity, spores are released, which are carried by wind and raindrops to cause infections. The pathogen can affect apple and pear trees, as well as strawberries, cherries, and highbush blueberries. In the case of apples and pears, the occurrence of anthracnose symptoms during storage often coincides with reaching the consumable ripeness. Moreover, excessively high temperatures in cold storage and during commercial handling stimulate the faster development of decay spots. In recent years, the severity of the disease in central Poland has not been high; however, in 2018, apple infection increased.

Brown rot of apples, known as moniliosis, is a disease caused by fungi of the *Monilinia* genus. This disease affects both pome fruit trees like apple trees and stone fruit trees. It particularly affects plums and apricots. The disease has two forms: a summer form that occurs in orchards and the second one - which becomes evident during storage. The harm caused by the disease lies in the rotting of the fruits.

Bull's eye rot of apples and pears is a disease caused by fungi of the *Neofabraea spp.* genus. These pathogens induce two types of symptoms: cankers on the bark of apple trees and the "eye rot" of apples and pears during storage. Bark cankers manifest as elliptical necrotic lesions on the tree branches. On the dead bark, elevations can be seen, which are clusters of conidial spores. Fungal spore production on dead twigs, short shoots, and canker lesions serves as a source of infection for the fruits.

Powdery mildew is the collective term for a group of diseases caused by fungi affecting the above-ground parts of plants. These fungi are highly specialized and often target specific plant species. Consequently, different fungal species causing the same disease can be found in various crops. For instance, roses are generally affected by the fungus *Podosphaera pannosa,* while grasses and cereals are affected by *Erysiphe graminis* or *Blumeria graminis. Uncinula necator* affects grapes, *Erysiphe heraclei* affects carrots, and *Podosphaera leucotricha -* apples. These fungi can induce symptoms on all above-ground parts: leaves, stems, fruits, and flowers. Beyond vegetable and ornamental crops, powdery mildew is also present in berry fruit cultivation. Despite the fact that different plant pathogenic fungi attack nearly every crop, a common feature of powdery mildews is the appearance of white, powdery spots, most commonly on the upper side of leaves. Typically, these spots start to appear on the lower parts of the plant. As the fungus spreads, it can cover the entire leaf surface, causing yellowing and ultimately drying out. In cases of severe infection, symptoms may also manifest on the lower side of the leaf and, depending on the species, other above-ground parts of the plant.

Fire blight is one of the most destructive diseases of apple and pear trees. It has been present in Poland for over 50 years and causes economic damage annually, particularly in orchards. It has been detected in almost all regions where fruit trees are cultivated. However, long-term observations indicate significant irregularity in its occurrence, both in terms of its severity in regions where it has been recorded for years, and its unexpected appearance in areas where it has never been detected before. The variability in the occurrence of fire blight is mainly associated with the course of atmospheric conditions that affect the survival of its causative agent, the bacterium *Erwinia amylovora,* within infected plant organs. The range of affected host plants is broad, encompassing over 130 species, primarily from the rose family. In addition to apple and pear trees, other plants affected by fire blight in Poland include hawthorn, quince, cotoneaster, fire thorn, rowan, and serviceberry.

On the other hand, soft rot is caused by bacteria from the species *Pectobacterium carotovorum,* which can lead to massive, soggy decay of roots or tubers either at the end of the vegetation period or during storage. The decay of roots is accompanied by the characteristic unpleasant smell of rotting fish associated with this disease. During the growing season, small watery spots are visible on the affected roots and tubers, which turn brown and enlarge in size. The tissue in these areas becomes soft, rots, and transforms into a semi-liquid, slimy, foul-smelling mass. The intensity of symptoms increases during storage, especially on mechanically damaged roots and tubers. The development of bacteria is favored by high temperatures and high humidity in storage facilities. Plants become infected in field conditions, during periods of prolonged soil moisture. The bacteria spread extensively in the soil where infected plant residues remain. In most cases, infection occurs during the transport and storage of wet and mechanically damaged roots and tubers. On fruits, wet rot can also be caused by fungi from the *Penicillium spp.* genus, such as *Penicillium expansum.*

There are known plant protection agents based on microorganisms in patent literature and scientific publications for use in plant cultivation. For instance, patent application PL418852 A1 reveals a biopreparation for protecting plant leaves against gray mold. This biopreparation consists of water (95%), humic acids (4%), and conidial spores of fungal strains *Trichoderma atroviride* Tr-43 and Tr-53, *Trichoderma harzianum* WT17AJ, and *Trichoderma asperellum* WT9, in a minimum quantity of 10⁶ colony-forming units per 1 ml of the preparation. This biopreparation forms the basis for a composition of a plant protection agent against gray mold on the leaves.

From the patent description PL238148 B1, a biopreparation for plant protection against bacterial infections, such as those caused by pectinolytic bacterial pathogens of the *Pectobacterium* and *Dickeya* genera (soft rot), is known. This biopreparation is in a powdered formulation containing diatomaceous earth or kaolin as a carrier, along with additives such as methylcellulose, β-cyclodextrin, sodium lignosulfonate, and KH₂PO₄. As the biologically active agent, it contains a lyophilized culture of a selected deposited strain of antagonistic bacteria, such as *Serratia plymuthica* strain A294 deposited under number B/00143, or *Serratia rubidaea* strain H469 deposited under number B/00142, or *Serratia rubidaea* antagonistic strain H440 deposited under number B/00141, or *Enterobacter amnigenus* antagonistic strain A167 deposited under number B/00145, or *Rahnella aquatilis* antagonistic strain H145 deposited under number B/00144, or a mixture of these five antagonistic bacterial strains.

From the patent description PL236445 B1, a novel mixture of antagonistic bacteria for plant protection is known. This mixture contains at least two strains selected from among the strains: *Serratia plymuthica* strain A294, *Enterobacter amnigenus* strain A167, *Rahnella aquatilis* strain H145, *Serratia rubidaea* strains H440 and H469. These strains were deposited in the Polish Collection of Microorganisms (PCM) at the Institute of Immunology and Experimental Therapy of the Polish Academy of Sciences, named after Ludwik Hirszfeld, in Wroclaw, on December 1, 2017. The patent also encompasses the application of the aforementioned mixture of antagonistic bacteria for protecting plants against pectinolytic bacterial infections, as well as the method of applying these antagonistic bacteria. According to this method, the mixture can be applied through spraying, fogging, plant dusting, soaking plants during storage, in greenhouse, field, and hydroponic conditions, where the antagonistic bacteria suppress disease symptoms caused by pectinolytic bacteria at temperatures ranging from 7°C to 40°C.

In the publication "Perspectives on the use of bacteria in the protection of apple and pear against fire blight (Erwinia amylovora)" authored by A. Mikiciński and P. Sobiczewski, and published in Progress in Plant Protection (2018), Volume 58, Issue 1, pages 68-75, the protection of plants against fire blight involves the integration of various methods aimed at preventing infection and managing the disease. Among chemical plant protection agents, copper-based preparations are almost exclusively registered for combatting fire blight in Poland. However, various limitations associated with their use have driven an increased interest in alternative methods, including biological approaches involving bacteria. The publication highlights that naturally occurring bacteria with antagonistic effects against the causative agent of fire blight have been identified in natural conditions. These bacteria possess the ability to protect plants from the disease. Research conducted in various countries has shown that the most effective species for biological control include *Pantoea agglomerans, Pseudomonas fluorescens, Bacillus subtilis,* and *Lactobacillus plantarum.* Extensive research on the application of various strains of *P. agglomerans* (formerly classified as *Erwinia herbicola)* for combating fire blight was conducted in the 1980s under the guidance of Professor S. V. Beer at Cornell University in New York state. This research is described in the publication: S. V. Beer, J. R. Rundle, titled "Suppression of Erwinia amylovora by Erwinia herbicola in immature pear fruits," published in Phytopathology (1983), Vol. 73, No. 9, pp. 1346-1346. Introducing a suspension of bacteria onto apple blossoms one day before or three days after inoculation with E. *amylovora* showed that 52% and 25% of flowers, respectively, remained externally healthy. Similar efficacy (52% of symptom-free flowers) was observed after treatment with streptomycin, as described in another publication by S. V. Beer and colleagues: "Control of fire blight by non-pathogenic bacteria" published in Phytopathology (1980), Vol. 70, No. 5, pp. 459-459.

Of particular note is the strain C9-1, isolated from canker on an apple tree trunk in Michigan. Initially identified as *P. agglomerans* (C. A. Ishimaru et al., "Multiple antibiotic production by Erwinia herbicola," Phytopathology, (1988), 78(6), 746-750), it was subsequently reclassified as P. *vagans* (C. L. Brady et al., "Pantoea vagans sp. nov., Pantoea eucalypti sp. nov., Pantoea deleyi sp. nov. and Pantoea anthophila sp. nov.", Int J Syst Evol Microbiol (2009), 59:2339-2345). Treating apple and pear trees with a suspension of the C9-1 strain during the flowering period reduced the occurrence of fire blight by 50% to 80%, showing similar effectiveness to treatment with streptomycin sulfate. Due to these promising results, the United States Environmental Protection Agency initiated efforts towards its commercialization, as described in the publication by K. B. Johnson and V. O. Stockwell, titled "Biological control of fire blight. Fire blight: the disease and its causative agent, Erwinia amylovora", published by CABI Publishing (2000), Oxon, United Kingdom, 319-337. Based on this strain, the first biopesticide against fire blight was developed, named BlightBan C9-1.

As revealed by the conducted analysis of the state of the art, known methods of biological plant protection against bacterial and fungal diseases, as well as preparations based on the potential of microorganisms, often offer a relatively narrow scope of action, limited to a small spectrum of targeted pathogens. Typically, this may involve only one pathogen. However, in practice, it is quite common for plants to be simultaneously affected by several pathogens. In such cases, using available solutions often requires the use of additional treatments to combat the other bacterial or fungal pathogens present on the plants at that moment, including synthetic plant protection agents. This leads to increased costs for plant protection and contributes to excessive chemical intervention in the natural environment. Unexpectedly, this problem has been solved in the current invention.

The objective of this invention is to isolate new strain- of bacteria belonging to the *Pantoea spp.,* with a significantly broader spectrum of activity than the currently known and used strains. The utilization of the strain could offer a solution to the inconveniences posed by the known methods in comprehensively protecting various plant species, including vegetables, fruits, and ornamental plants, against simultaneous attacks by different fungal and/or bacterial diseases.

The substance of the invention lies in the strain *Pantoea agglomerans* T16/8, which has been deposited in the Polish Collection of Microorganisms PCM at the Institute of Immunology and Experimental Therapy of the Polish Academy of Sciences in Wroclaw, under the number B/00272, on November 26, 2019.

The substance of the invention also includes the use of strain T16/8 of *Pantoea agglomerans* deposited in the Polish Collection of Microorganisms PCM at the Institute of Immunology and Experimental Therapy of the Polish Academy of Sciences in Wroclaw, on November 26, 2019, selected from the strain *Pantoea agglomerans* T16/8 deposit number B/00272, as a means for protecting plants against diseases caused by bacterial and/or fungal pathogens.

The most beneficially, the strain *Pantoea agglomerans* T16/8 are applied in the form of an aqueous suspension to the plant surface, through spraying, soaking of plants or their parts in storage conditions, under cover and in field conditions, with the cell count of the strain in the suspension being at least 10⁷ colony-forming units/ml.

The strain *Pantoea agglomerans* T16/8, which are the subject of the invention, stand out due to their wide spectrum of activity. This strain exhibits antagonistic activity against a variety of plant pathogens, both fungal and bacterial, responsible for diseases such as brown rot, gray mold, powdery mildew, alternariosis leaf spot, anthracnose, brown rot, fire blight, and blue mold. Furthermore, this strain can be used to protect numerous plant species, including apple, pear, raspberry, strawberry, highbush blueberry, grapevine, carrot, cabbage, lettuce and celery. The range of susceptible plant species to gray mold pathogen is reported by Borecki ("Szkodniki i choroby roślin sadowniczych" ("Pests and Diseases of Fruit Plant"), 3rd edition, PWRiL, 1983) to be over 100 species. As stated by Leski ("Szkodniki i choroby roślin warzywnych" ("Pests and Diseases of Vegetable Plant"),, PWRiL, 1985), white rot pathogen affects numerous herbaceous plants while soft rot-causing agent (bacteria), attacks most of crop plants including all vegetables.

The application of strains of the *Pantoea spp.* genus, according to the invention, reduces the costs of plant protection by reducing the amount of preparations needed to combat various bacterial or fungal pathogens occurring simultaneously on plants during the same period. This, in turn, helps to avoid excessive chemical treatment of plants. Conducted studies have demonstrated that the *Pantoea agglomerans* T16/8 strain exhibit exceptionally strong antagonistic activity against at least 10 plant fungal and/or bacterial pathogens that were tested under laboratory and field conditions.

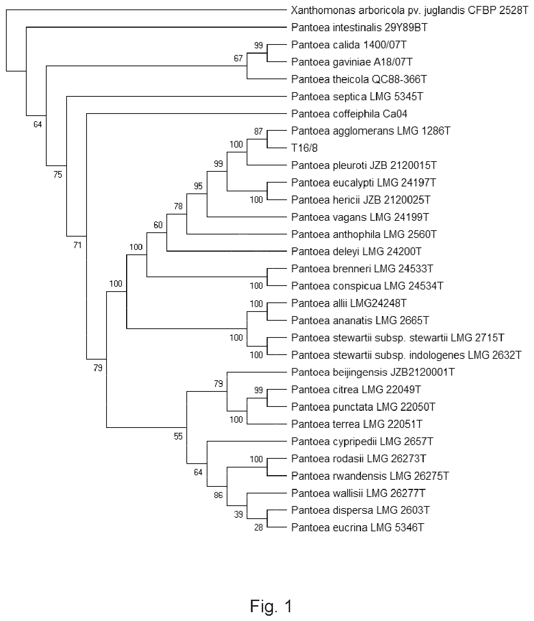

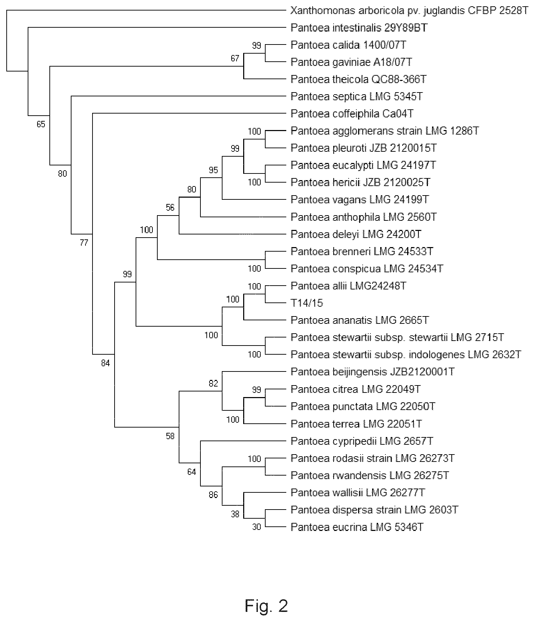

The characteristics of *Pantoea agglomerans* T16/8 strain, as well as the results of *in vitro* or *in vivo* studies confirming their effectiveness in exemplary research conducted in apple, carrot, cabbage, lettuce, celery, and grapevine crops, are presented in the following examples. The phylogenetic relationships among all *Pantoea spp.* species and the *Pantoea agglomerans* T16/8 strain are depicted in Figure 1, based on a combined set of gene sequence data including *atpD, gyrB, infB,* and *rpoB.*

These example should not be regarded as limiting the substance of the solution or restricting the range of the protective invention, as they merely serve as illustrations.

### Example 1

### Identification of Pantoea agglomerans T16/8 strain

The *Pantoea agglomerans* T16/8 strain was deposited in accordance with the Budapest Treaty at the Polish Collection of Microorganisms (PCM), Institute of Immunology and Experimental Therapy, Polish Academy of Sciences, named after Ludwik Hirszfeld, in Wroclaw. The deposit was made on November 26, 2019. The deposited strain was assigned the number B/00272.

### Isolation and Colony Morphology of Pantoea agglomerans T16/8 strain

The bacteria were isolated from the surface of strawberry leaves at the beginning of the growing season. Cut leaves (a few pieces) were placed in 500 ml Erlenmeyer flasks containing approximately 200 ml of sterile distilled water with the addition of one drop of Tween 80, and then shaken for 30 minutes. The washings was transferred to sterile plastic falcon tubes. The supernatant was poured off, and the bacteria were isolated from the sediment by plating them onto NAS medium (2.3% nutrient agar with the addition of sucrose to a final concentration of 5%) in 90 mm Petri dishes. *Pantoea agglomerans* T16/8 colonies, after 24 hours of incubation at 25°C, formed strongly spreading bright-yellow, optically dense, circular colonies with a diameter of 2 mm. After another day, their diameter reached 3-4 mm. Further incubation of the strain (25°C) caused strong spreading on the surface of the NAS medium, which could lead to the merging of adjacent colonies and even filling the entire surface. Over time, the color of the colonies remained consistently bright-yellow.

### Storage of T16/8 Strain

The *Pantoea agglomerans* T16/8 strain is stored at a temperature of -80°C in glycerol in PBS buffer (0.27% Na₂HPO₄, 0.04% NaH₂PO₄, 0.8% NaCl). To deposit the strain, it was plated onto NAS medium (in 90 mm Petri dishes), and after 24 hours of incubation at 25°C, it was washed with sterile buffer (1 ml). 0.8 ml of the suspension was taken and transferred to a sterilized Eppendorf tube along with 0.2 ml of glycerol. The bacterial strain T16/8 suspension was vigorously mixed (vortexed) to ensure thorough mixing and was then placed in a cryovial and transferred to a low-temperature freezer at -80°C.

### Species Identification of Pantoea agglomerans T16/8 Strain

The species identification of the *Pantoea agglomerans* T16/8 strain was determined through phylogenetic analysis based on the sequences of:
A. the gene encoding 16S rRNA,
B. fragments of housekeeping genes *atpD, gyrB, infB,* and *rpoB,*
C. analysis of the strain's genome sequence.

For the phylogenetic analyses (A, B), DNA was isolated from a 48-hour culture of bacteria potentially useful for biological protection in NSA medium (2.3% Difco Nutrient Agar; 5% sucrose), using the Genomic Mini kit for bacterial DNA isolation (A&A Biotechnology, Poland) according to the provided manufacturer's instructions. The isolation material was prepared by suspending a pure isolate colony in 100 µl of Tris buffer (10 mM Tris-HCl, pH 8.5). To the resulting suspension, 200 µl of universal lysis buffer LT (0.25% SDS, 0.2 M NaOH) and 20 µl of Proteinase K solution were added. The mixture was thoroughly mixed and incubated at 37°C for 20 minutes. The sample was then transferred to 75°C and incubated for additional 5 minutes. The mixture was then vortexed for 20 seconds on a Vortex mixer and then centrifuged for 3 minutes at 15000 rpm. The obtained supernatant was applied to a mini column for genomic DNA purification. The column was centrifuged for 1 minute at 15000 rpm. In the next step, 500 µl of elution buffer A1 was added to the column, and the column was centrifuged again for 1 minute at 15000 rpm. The mini column was transferred to a new 2 ml test tube, and then 400 µl of elution buffer A1 was added to the column. The sample was centrifuged for 2 minutes at 15000 rpm. The dried mini column was then placed in a new 1.5 ml test tube. For elution, 100 µl of Tris buffer preheated to 75°C was applied to the mini column bed. The sample was incubated for 5 minutes at room temperature and then centrifuged for 1 minute at 15000 rpm. For the PCR reaction, 3 µl of the obtained DNA was taken.

### A. Analysis of the 16S rRNA gene sequence.

For the PCR reaction, primers fD1 - 5' AGAGTTTGATCCTGGCTCAG 3' and rP2 - 5' ACGGCTACCTTGTTACGACTT 3' were used, as described in the publication by Weisburg et al., titled "16S ribosomal DNA amplification for phylogenetic study", Journal of Bacteriology (1991), 173, 697-703. The PCR reactions were carried out in a Biometra^{®} T3000 thermocycler. The following amplification reaction conditions were used: initial denaturation at 95°C for 4 minutes, 35 cycles at 95°C for 45 seconds, 55°C for 45 seconds, and 72°C for 1 minute 30 seconds. The final extension step was performed at 72°C for 10 minutes. The resulting PCR product was separated on a 1.5% agarose gel in 0.5 x TBE buffer. To determine the size of the obtained product, a 1 µl sample of O'Gene RulerTM 100 bp Plus DNA Ladder (#SM1153) from Fermentas was used as a marker. The reaction product was sequenced at Genomed S.A. (Warsaw). The obtained sequences for each isolate were assembled using the SeqMan software of the Lasergene package (DNASTAR Inc., Madison, Wisconsin), and they were compared with sequences deposited in the NCBI GenBank (http://www.ncbi.nlm.nih.gov) and the EzTaxon database (https://www.ezbiocloud.net/) to find the closest matches.

The identification based on the 16S rRNA gene sequence allowed the classification of the studied isolates into the genus *Pantoea.*

### B. Analysis of sequences of core metabolism genes fragments.

For the phylogenetic analysis, fragments of 4 core metabolism genes were used: *atpD, gyrB, infB,* and *rpoB.* First, gene fragments were amplified using specific primers for the infB genes. The amplification conditions were applied as described in the publications: C. Brady et al., "Phylogeny and identification of Pantoea species associated with plants, humans and the natural environment based on multilocus sequence analysis (MLSA)", Systematic and Applied Microbiology (2008), 31(6-8), pp.447-460, and L. Diancourt et al., "Multilocus sequence typing of Klebsiella pneumoniae nosocomial isolates" (2005), J Clin Microbiol 43: 4178-4182. PCR reactions were performed in a Biometra^{®} T3000 thermocycler. Additionally, gene sequences of *atpD* and *gyrB* were extracted from the genome of the T16/8 strain. The sequences of all four genes were combined into a single sequence and compared with sequences of the same genes from type strains of other *Pantoea* species deposited in the NCBI database. For the phylogenetic analysis, all gene sequences were considered (accession numbers are provided on the phylogenetic trees). Maximum Likelihood (ML) trees based on the combined sequences of the four analyzed genes were generated in the MEGA X program using a parametric and evolutionary model recognized as the best substitution model for all sequences of each gene, individually calculated by the program, and with 500 bootstrap replications.

The phylogenetic analysis and the resulting Maximum Likelihood (ML) tree, constructed based on the combined sequences of the core genes *atpD, gyrB, infB,* and *rpoB,* demonstrated that the T16/8 strain is most closely related to the *Pantoea agglomerans* species. The Maximum Likelihood tree presented in the figure, based on the Tamura-Nei model, illustrates the phylogenetic relationships between the T16/8 strain and all *Pantoea* spp. species using the combined set of sequence data from the *atpD, gyrB, infB,* and *rpoB* genes. A discrete Gamma distribution was used to model evolutionary rate differences between sites (5 categories (+G, parameter = 0.4052)). The rate variation model allowed some sites to be evolutionarily invariable ([+I], 24.38% sites). Bootstrap values (expressed as percentage values from 500 replications) are indicated at nodes. The letter "T" following the strain names indicates that a given strain is a typical strain of the species.

### C. Genome Sequencing and Analysis

For the genome sequencing of the *Pantoea agglomerans* T16/8 strain, DNA was isolated using the method described by S.M. Aljanabi and I. Martinez in their publication "Universal and rapid salt-extraction of high quality genomic DNA for PCR-based techniques" (1997), Nucleic Acids Res. 25(22), 4692-3. Libraries with short inserts (approximately 350 bases) were prepared for sequencing on the MiSeq device (Illumina), as well as libraries for sequencing on the MiniON device (Oxford Nanopore Technologies). A total of 3,495,070 paired-end reads with short inserts (insert size 300-500 bp) were obtained from the MiSeq (Illumina) sequencing, and 76,550 long reads were obtained from the MinION sequencing. All reads were used for *de novo* genome assembly (Genomed SA, Warsaw, Poland). The *de novo* assembly was performed using the Unicycler software version 0.4.7. The obtained sequences of all genetic elements were annotated using the RAST program.

The resulting high-quality genome of the T16/8 strain revealed that its genomic material consists of a chromosome (T16/8_chrom size: 4,099,784 bp) and three plasmids (pT16/8_1 size: 547,479 bp, pT16/8_2 size: 181,799 bp, pT16/8_3 size: 75,138 bp).

### Average Nucleotide Identity (ANI) Calculation

The Average Nucleotide Identity (ANI) was calculated for the genome of the *Pantoea agglomerans* T16/8 strain and the type strains of related species: *Pantoea agglomerans* DSM3493T, *Pantoea allii* LMG24248T, *Pantoea ananatis* LMG2665T, and *Pantoea vagans* LMG24199T. This calculation was performed using the JSpecies algorithm (JSpeciesWS; http://jspeciesWS; http://jspeciesWS; .ribohost.com/jspeciesws/). Values of ANIMUMmer (ANIm) and ANI-Blast (ANIb), between two strains should be higher than 95% to classify strains within the same species according to the criteria described in the publications by K.T. Konstantinidis et al., titled "Toward a more robust assessment of intraspecies diversity, using fewer genetic markers" (Appl. Environ. Microbiol. 2006, 72, 7286-7293) and M. Richter and R. Rossello-Mora, titled "Shifting the genomic gold standard for the prokaryotic species definition", Proc. Natl. Acad. Sci. U. S. A., 2009, 106, 19126-19131. The ANI analysis confirms the classification of the T16/8 strain within the *Pantoea agglomerans* species, as the ANI value between the genome of the *Pantoea agglomerans* T16/8 strain and the genome of the typical strain of *Pantoea agglomerans* is higher than 95% (Table 1).

**Table 1**

| ANIb and Percentage Similarity | |
|---|---|
| | T16/8 |
| T16/8 | * |
| *Pantoea agglomerans* DSM3493^{T} | **98.49** |
| *Pantoea allii* LMG24248^{T} | 78.47 |
| *Pantoea ananatis* LMG2665^{T} | 78.37 |
| *Pantoea vagans* LMG24199^{T} | 91.26 |

The results of the conducted analysis have shown that the strain belongs to the following species: *Pantoea agglomerans.*

### Pathogenic Properties

The pathogenic properties of *Pantoea agglomerans* T16/8 strain were tested on:
- tobacco - to detect hypersensitivity reactions
- potato tubers - for testing pectinolytic properties
- plant species mentioned in the literature as susceptible to bacteria of the species *P*. *agglomerans:* alocasia, onion, adzuki bean, corn, wheat, pea, perennial ryegrass. In none of the tests did the *Pantoea agglomerans* T16/8 strain exhibit pathogenic properties.

### Example 2

Application of *Pantoea agglomerans* T16/8 Strain for Plant Protection Against ***Sclerotinia sclerotiorum-caused* white rot**
*In vitro* antagonistic properties of *Pantoea agglomerans* T16/8 strain against *Sclerotinia sclerotiorum* were evaluated. First, the inhibition of *Sclerotinia sclerotiorum* growth by *Pantoea agglomerans* T16/8 strain was examined. The experiments were conducted on PDA medium (potato dextrose agar), using co-cultures of *Pantoea agglomerans* T16/8 strain along with *Sclerotinia sclerotiorum,* and the inhibition of the fungal growth was observed. The zone of inhibition of *Sclerotinia sclerotiorum* growth by the T16/8 strain, after 5 and 7 days, is presented in Table 2.

**Table 2**

| Strain | Zone of growth inhibition after 5 days [mm] | Zone of growth inhibition after 7 days [mm] |
|---|---|---|
| Control | 0 | 0 |
| T16/8 | 2 | 0 |

The numerical values presented in Table 2 indicate the radius from the edge of the colony to the edge of the growth inhibition zone in millimeters. The inhibition of pathogen growth by the tested strain indicates the ability of T16/8 strain to produce substances with fungicidal or fungistatic properties.

The subsequent experiment was conducted in laboratory conditions on leaves of cabbage and carrot. After causing damage using a preparation needle, cabbage leaves and carrot fragments were soaked for 5 minutes in aqueous suspensions, one containing *Pantoea agglomerans* T16/8 strain strain, at a concentration of 10⁷CFU/ml. After 2 hours, mycelial disks from 7-day cultures of *Sclerotinia sclerotiorum* were placed at the site of damage. Control samples consisted of cabbage and carrot leaves soaked in distilled water and inoculated with *Sclerotinia sclerotiorum.* Inoculated cabbage and carrot leaves were placed in trays on moist mats covered with grids. The trays with the leaves were then placed inside plastic bags to maintain increased humidity. After 3 and 5 days of incubation at a temperature of 25°C, the length of infectious lesions was measured. The test was conducted in 6 repetitions, with bacterial strain being tested on 6 plant fragments (cabbage leaves and carrot fragments). Among the 426 environmental isolates tested, *Pantoea agglomerans* T16/8 strain exhibited some of the highest effectiveness in inhibiting the growth of S. *sclerotiorum*: 62.5% - 81%.

Subsequently, an assessment of the *in vivo* antagonistic properties of *Pantoea agglomerans* T16/8 strain against *Sclerotinia sclerotiorum* was conducted in greenhouse and field experiments.

The impact of T16/8 bacterial strain on limiting the development of *Sclerotinia sclerotiorum-caused* white rot was evaluated in greenhouse cultivation of lettuce. For this purpose, lettuce seedlings of the variety 'Królowa Majowych' were transplanted into 0.5-liter P9 pots filled with peat substrate, which were artificially infected with *Sclerotinia sclerotiorum.* The pathogen inoculum was prepared using oatmeal agar. The plants were placed on window sills in the greenhouse and cultivated for 1.5 months. During this period, the lettuce was watered three times with aqueous suspensions of T16/8 strain, 50 ml per pot, with the bacterial cell concentration in the suspension being 10⁷ CFU/ml. The first application was performed before transplanting the lettuce into the infected substrate. Subsequent applications were conducted at 10-day intervals. The uninfected control consisted of plants growing in clean peat substrate without the pathogen, while the infected control involved lettuce grown in substrate inoculated with *S. sclerotiorum* and watered with distilled water. The standard (comparative) treatment utilized a commercial product known as Contans WG. The experiment was conducted in 4 repetitions, with 5 plants in each (a total of 20 plants per combination). In the central part of 90 mm diameter Petri dishes, 5 mm diameter PDA agar disks overgrown with mycelium were placed. The pathogen inoculum - *S. sclerotiorum -* was prepared. The dishes were incubated for 10 days at 25°C. Subsequently, the agar was homogenized (using a homogenizer H500, Pol-Eko Aparatura, Poland) with distilled water (150 ml of water per dish) for 5 minutes. The resulting suspension was thoroughly mixed with peat substrate (TS1 Klasmann, Poland) in a ratio of: contents of 1 dish to 1 liter of substrate. The infected peat was incubated for 7 days in the greenhouse, inside bags with small openings. After a week of incubation, the experiment was set up.

On the day of each treatment and one week and two weeks after the last watering of lettuce, the degree of plant infection was determined using an 8-point scale: (0-0%; 1-1%; 2-5%; 3-15%; 4-25%; 5-50%; 6-75%; 7-100%). The obtained research results were statistically analyzed using analysis of variance. To determine differences between means, the Duncan test was used, with a significance level of α=0.05. The effectiveness of the preparations was evaluated using the Abbott's formula. The impact of the tested bacteria on limiting *Sclerotinia sclerotiorum*-caused white rot in lettuce is presented in Table 3.

**Table 3**

| Tested strain/reference product | % of Infection | % of Lettuce infection | Protection efficacy, [%] | % of Lettuce infection | Protection efficacy [%] |
|---|---|---|---|---|---|
| | 1st assessment | 2nd assessment | | 3rd assessment - at the end of the experiment | |
| Uninoculated control | 0.0 | 0.0 | - | 0.0 | - |
| Inoculated control | 3.4 | 9.6 | - | 45.9 | - |
| T 16/8 | 0.0 | 3.5 | 63.5 | 24.3 | 47.0 |
| Contans WG | 0.0 | 0.9 | 90.6 | 10.8 | 76.4 |

An evaluation of the effectiveness of T16/8 strain in limiting *Sclerotinia sclerotiorum*-caused decay (white rot) on celery roots was also conducted in a pot experiment. For this purpose, 'Talar' variety celery plants were transplanted into 12 cm diameter pots filled with peat substrate previously infested with *S. sclerotiorum* and placed in a greenhouse (plant growth stage: BBCH 13). There were 5 plants per plot. During the celery growth period, 4 plant protection treatments were carried out by watering the plants. A total of 20 ml of working solution was used per plant, with one containing *Pantoea agglomerans* T16/8 strain strain, at a concentration of 10⁷ CFU/ml. The treatments were applied every 7 days.

An untreated control (non-inoculated) consisted of plants grown in pure peat substrate without the pathogen. The inoculated control (inoculated) consisted of lettuce grown in substrate infested with *S. sclerotiorum* and watered with distilled water. A commercial product Contans WG was used as a standard (comparative) measure. The first treatment was performed 7 days before transplanting the plants into pots, i.e., celery seedlings growing in multiplates. The infection of celery plants was induced artificially. The substrate was infected with *S. sclerotiorum* at a ratio of 1:1, i.e., 1 liter of substrate to 1 petri dish of *S*. *sclerotiorum* mycelium. Observations of the percentage of leaf infection in celery by the causative agent of *Sclerotinia* rot were conducted on 7 randomly selected leaves, at the onset of the first disease symptoms. The assessment was done using an 8-point scale, where 0 = no disease symptoms, 1° - up to 1% of leaf area affected, 2° - 2 to 6% of leaf area, 3° - 7 to 15% of leaf area, 4° - 16 to 25% of leaf area, 5° - 26 to 50% of leaf area, 6° - 51 to 75% of leaf area, 7° - 76 to 100% of leaf area with *Sclerotinia* rot symptoms. Measurements of plant height in centimeters (to the base of the highest leaf and whole leaves) were taken. Leaf color was also determined on an 11-point scale, with 5 representing the control, 0-4 representing light green, pale, yellowing leaves, and 6-10 representing dark green to very dark green leaves. Phytotoxicity symptoms were assessed using the EPPO PP 1/135(4) (2014) scale, ranging from 0 to 100%, where 0 indicated no signs of damage, and 100 indicated complete plant destruction. The results were statistically analyzed using analysis of variance. Differences between means were assessed using the Duncan test at a significance level of 5%. The effectiveness of the tested agents was calculated using the Abbott's formula.

The assessment of the effectiveness of the tested microorganism strain in protecting celery against *Sclerotinia* rot is presented in Table 4.

**Table 4**

| Combinations | | White rot *- Sclerotinia sclerotiorum* | | | | | | | |
|---|---|---|---|---|---|---|---|---|---|
| | | Average % of leaf infection | | | | Efficacy [%] | | | |
| | | Observation date | | | | Observation date | | | |
| | | 1st assess ment | 2nd assessm ent | 3rd assess ment | 4th assess ment | 1st assessm ent | 2nd assessm ent | 3rd assess ment | 4th assessm ent |
| 1 | Uninoculated control | 0 | 0 | 0 | 0 | - | - | - | - |
| 2 | Inoculated control | 2.64 | 8.57 | 18.43 | 33.06 | - | - | - | - |
| 3 | T16/8 | 0.14 | 0.86 | 2.97 | 13.53 | 94.7 | 89.9 | 83.9 | 59.1 |
| 4 | Contans WG | 0.14 | 0.97 | 3.58 | 13.95 | 94.7 | 88.7 | 80.6 | 57.8 |

The results of both experiments conducted for lettuce and celery (Table 3, Table 4), carried out under provocative conditions involving artificial substrate inoculation, clearly indicate the high effectiveness and thus the high utility of the tested strain T16/8 for combating *Sclerotinia* rot. The achieved effectiveness results of the tested strain were at least as effective as the recognized standard, represented by the Contans WG product. Additionally, a positive impact of the strain on the growth and development of celery plants was observed, as presented in Table 5.

**Table 5**

| Combinations | | Height of celery plants (cm) | | |
|---|---|---|---|---|
| | | Observation date | | |
| | | 1st assessment | 2nd assessment | 3rd assessment |
| | | Measurement from the base to the tip of the highest leaf | | |
| 1 | Uninoculated control | 7.53 | 8.78 | 10.55 |
| 2 | Inoculated control | 7.43 | 8.9 | 10.35 |
| 3 | T16/8 | 7.83 | 8.8 | 10.43 |
| 4 | Contans WG | 7.25 | 7.38 | 8.6 |

### Example 3

Application of *Pantoea agglomerans* T16/8 strain for plant protection against **gray mold** caused by ***Botrytis cinerea.***

An assessment of the *in vitro* antagonistic properties of *Pantoea agglomerans* T16/8 strain against *Botrytis cinerea* was conducted. Firstly, the limitation of *Botrytis cinerea* growth *by Pantoea agglomerans* T16/8 strain was investigated. The experiments were carried out on PDA medium (potato dextrose agar), involving co-culturing of *Pantoea agglomerans* T16/8 strain with *Botrytis cinerea,* and the inhibition of the latter's growth was observed. The zone of inhibition of *Botrytis cinerea* growth by T16/8, after 5 and 7 days, is presented in Table 6.

**Table 6**

| Strain | Zone of Inhibition after 5 days [mm] | Zone of Inhibition after 7 days [mm] |
|---|---|---|
| Control | 0 | 0 |
| T16/8 | 5 | 0 (n.g.) |

The numerical values presented in Table 9 indicate the radius from the edge of the colony to the edge of the growth inhibition zone in millimeters. The inhibition of pathogen growth by the tested T16/8 strain indicates their capability to produce substances with fungicidal or fungistatic properties. The abbreviation "n.g." stands for "no growth on surface" - the fungal growth reached the bacterial border but did not extend onto its surface. This suggests a mechanism different from antibiosis, including parasitism or toxins, which might not diffuse into the microbiological medium but remain trapped within the microbial growth.

An evaluation of the in vitro antagonistic properties of *Pantoea agglomerans* T16/8 strain against *Botrytis cinerea* was performed on apple blossoms. One-year-old apple trees of the 'Najdared' variety were stored in a regular refrigerator at a temperature of 2-3°C and relative humidity of 98%. Subsequently, they were planted in 24 cm diameter pots with a mixture of peat and soil in a 1:1 ratio. The planted trees were incubated in a greenhouse at a temperature of 20°C for about 2 weeks. After this period, the flower buds started to develop. On each tree, 20 fully bloomed flowers were left, while the rest were removed to minimize experimental errors. Each prepared plant represented a single combination. Using a sprayer, bacterial suspensions were applied to the flowers, and distilled water was applied to the control combination. The suspensions contained *Pantoea agglomerans* T16/8 strain, at a concentration of 10⁷ CFU/ml. After 24 hours, conidial spore inoculum of *B. cinerea* (Iz1) was applied to the flower buds at a concentration of 10⁶ spores/ml. An hour after the infection, plastic bags were placed over the flower buds to maintain high humidity levels beneficial for the development of the applied fungus. The following day, the bags were removed to avoid disrupting the natural physiological processes of the plants. After 5-7 days from the inoculation, individual flowers were carefully collected, avoiding contact with each other, and placed in Falcon tubes, which were then transported to the laboratory. The collected flowers were cut longitudinally at the base using a sterile scalpel, dividing them into two symmetrical halves. The plant material obtained was laid out on Petri dishes with a diameter of 90 mm containing PDA medium. The readings of the experiment were taken 24 hours after the flowers were placed on the medium. Selected bacterial strain were tested on the blossoms multiple times. The results of the study are presented in Table 7.

**Table 7**

| Strain | Pathogen | |
|---|---|---|
| | *Botrytis cinerea* Iz1 | Protection Efficacy [%] |
| Control | 100 | - |
| T16/8 | 9.0 | 10 |

The next experiment was conducted on apples. During the apple inoculation, two isolates of *B. cinerea* (Iz1 and Iz2) were used, which were obtained from diseased apples from different orchards. Spore suspensions of the fungus were prepared by rinsing 14-day-old cultures with sterile distilled water. Then, 250 ml Erlenmeyer flasks filled with 100 ml of the fungal suspension were placed on a shaker to disperse the spore clusters. After about 2 hours of shaking, the fungal suspensions were standardized using a Bürker chamber. The concentration of conidial spores was adjusted to 10⁶ spores/ml. In the conducted study, "Gala" apples from the Experimental Orchard were used. Immediately after harvest, the apples were stored in a regular refrigerator at a temperature of 2°C. The apples were pre-cleaned by rinsing in water. After drying, the skin surface was disinfected using cotton soaked in 95% ethanol. Four evenly spaced wounds with a diameter of 5 mm and a depth of 3 mm were made using a cork borer in the middle part of each fruit's circumference. Then, 30 µl of bacterial suspension was introduced into each wound. A suspension used was containing *Pantoea agglomerans* T16/8, at a concentration of 10⁷ CFU/ml. After 1 hour, 30 µl of spore suspension of the B. cinerea isolate Iz1 or Iz2 was added to each wound. The concentration of conidial spores was adjusted to 10⁶ spores/ml. In the control combination, 30 µl of sterile distilled water was introduced into the wounds, and after 1 hour, 30 µl of the spore inoculum of the respective B. *cinerea* isolate was added. The apples were stored for 4 days at room temperature (approximately 19°C) and relative humidity above 90%. The results of the study are presented in Table 8.

**Table 8**

| Strain | Name and isolate number of the fungal pathogen | | | |
|---|---|---|---|---|
| | *Botrytis cinerea* Iz1 concentration 10⁶/ml | Efficacy [%] | *Botrytis cinerea* Iz2 concentration 10⁶/ml | Efficacy [%] |
| Control | 31.1 | - | 35.0 | - |
| T16/8 | 9.0 | 71 | 11.3 | 68 |

The research was also conducted on grapes. Mature grapes of the "Solaris" variety were collected from the plantation, then sterilized by immersion in 50% ethanol and washed several times with sterile distilled water. After drying, the fruits were pierced with a sterile needle to a depth of about 1 mm and placed with the wound facing up on Petri dishes with a diameter of 20 cm, after prior immersion in aqueous suspensions of the tested bacteria. A suspension used was containing *Pantoea agglomerans* T16/8 at a concentration of 10⁹ CFU/ml. After 24 hours, inoculation was performed by applying drops of *B. cinerea* spore suspensions onto the created wounds using a pasteur pipette, with approximately 50 µl of the suspension. The control combination consisted of grape berries infected solely with the spore suspension of the pathogenic fungus B. cinerea. Each combination consisted of 60 berries (20 berries in 3 repetitions). The results were assessed according to the scoring scale: 0 - no symptoms of gray mold on the surface of the grape skin, 1 - 25%, 2 - 50%, 3 - 100% of the surface affected by the disease. The results of the study are presented in Table 9.Table 9

| Combination | Fruit damage on a rating scale | Efficacy [%] |
|---|---|---|
| Control | 1.86 | - |
| T 16/8 | 0.57 | 69.4 |

The obtained results confirm the effectiveness of the T16/8 strain in protecting grapes against gray mold.

Another experiment was conducted under laboratory conditions on cabbage and carrot leaves. After damaging with a preparatory needle, cabbage leaves and carrot fragments were soaked in aqueous suspensions for 5 minutes, containing *Pantoea agglomerans* T16/8, each at a concentration of 10⁷ CFU/ml. Then, after 2 hours, fungal mycelium disks from 7-day-old cultures of *Botrytis cinerea* were applied to the damaged areas. Control groups consisted of cabbage and carrot leaves soaked in distilled water and inoculated with *Botrytis cinerea.* The inoculated cabbage leaves and carrot fragments were placed in trays on moistened mats covered with mesh. The trays with cabbage leaves and carrot fragments were then placed inside plastic bags to increase humidity. After 3 and 5 days of incubation at a temperature of 25°C, the length of infectious spots was measured. The test was carried out in 6 repetitions, with each bacterial strain tested on 6 plant segments (cabbage leaves and carrot fragments). Among the 426 environmental isolates examined, the strain T16/8 showed one of the highest efficacies in inhibiting the growth of *B. cinerea.* - 45.8% - 67%.

Next, an assessment of the *in vivo* antagonistic properties of *Pantoea agglomerans* T16/8 strain against *Botrytis cinerea* was conducted in greenhouse and field experiments. The effectiveness of strain T16/8 in controlling gray mold on apples during fruit storage was evaluated. The biological efficacy of the tested bacteria in combating gray mold on apples was assessed after fruit storage by comparing the number of apples with disease symptoms in the experimental combinations (sprayed with aqueous suspensions of T16/8) and the control combination (unsprayed). In the studies, suspensions were used containing *Pantoea agglomerans* T16/8 strain at a concentration of 10⁷ CFU/ml. The research was conducted simultaneously and separately for each strain. The first treatments were performed during the flowering period, and subsequent ones in the pre-harvest period, 4, 3, 2, and 1 week before harvest, as well as "drenching," which involved dousing the fruits placed in crates with different aqueous suspensions of T16/8 strain immediately after harvest. The first evaluation was conducted after over 5 months of apple storage in a common refrigerator at a temperature of 2°C (1.8 - 2.2°C) and relative air humidity of 90 - 92%. The second evaluation was carried out after 7 days of apple storage at a temperature of 15-20°C. In each combination, 1000 pieces of fruit were evaluated (4 repetitions x 250 pieces per repetition), counting healthy apples and those with disease symptoms. The results were expressed as the percentage of affected fruit relative to all the apples evaluated in a given combination. The results were statistically analyzed using the analysis of variance method. Differences between means were evaluated using the Newman-Keuls test at a significance level of 5%. The effectiveness assessment results obtained from the conducted observations are shown in Table 10. Symbols A, B, C, D, E, F, G - denote application timings (A - full bloom, B - petal fall, C, D, E, F - respectively, 4, 3, 2, 1 week before harvest, G - "drenching" of fruits). Comparative studies were also conducted for commercial preparations Captan 80 WDG and Luna Experience 400 S.C.

**Table 10**

| Combination | Treatment dates/concentrat ion - dosage | I assessment after fruit storage in cold room | | II assessment after fruit storage at room temperature | |
|---|---|---|---|---|---|
| | | *Botrytis cinerea* | Efficacy [%] | *Botrytis cinerea* | Efficacy [%] |
| Control | - | 0.7 | - | 1.0 | - |
| Captan 80 WDG, Luna Experience 400 SC | ABC 1.9 kg | 0.4 | 42.9 | 0.7 | 30 |
| | E 0.75 | | | | |
| T16/8 | CDEFG 10⁷ | 0 | 100 | 0 | 100 |

An evaluation of the effectiveness of *Pantoea agglomerans* T16/8 strain in combating gray mold (*Botrytis cinerea*) on "Solaris" grape variety fruits harvested in the advanced ripening stage (BBCH 89) was also conducted. Protection of the grapes with the tested preparations began at the start of flowering, when 10% of the petals had fallen off. The assessment of the effectiveness of strain T16/8 in combating gray mold on grapes during fruit storage was performed. A water suspension containing one of the following was used: strain *Pantoea agglomerans* T16/8 at a concentration of 10⁷ CFU/ml. The first evaluation of gray mold occurrence was conducted immediately after fruit harvest, just before placement in cold storage. The second evaluation was performed after 40 days of storage in cold storage, and the third after 7 days of storage at 21°C. The results of each evaluation were presented as the percentage of affected berries. The effectiveness of the tested preparation was determined based on the comparison of the percentage of affected berries in the protected combinations compared to the control combination (untreated). The results were statistically analyzed using the analysis of variance method. Differences between means were evaluated using the Newman-Keuls test at a significance level of 5%. The results of the effectiveness assessment obtained from the conducted observations are shown in Table 11. Symbols A, B, C, D, E, F, G - denote application timings (A - beginning of flowering, B - full flowering phase, C - 70% of caps have fallen off, D - end of flowering phase, E - berries begin to touch each other, F - beginning of ripening, berries start to color, G - berries soften, H - berries ripe for harvesting). Comparative studies were also conducted for commercial preparations Serenade ASO, Luna Experience 400 S.C., Switch 62.5 WG).

**Table 11**

| Gray mold - grapevine 'Solaris' | | | | | | | |
|---|---|---|---|---|---|---|---|
| Combination | Treatme nt dates | I assessment immediately after harvest | | II assessment after 40 days of storage in cold storage | | III assessment after 7 days of storage at 21°C | |
| | | average % of infection | Efficacy [%] | average % of infection | Efficacy [%] | average % of infection | Efficacy [%] |
| Control | - | 29.6 | - | 77.0 | - | 84.4 | - |
| T16/8 | ABCDEF GH | 3.5 | 91.9 | 12.5 | 83.8 | 20.9 | 75.2 |
| T16/8 | BCEF | 5.2 | 82.4 | 12.8 | 83.4 | 26.0 | 69.2 |
| Serenade ASO | BCEF | 0.9 | 97.0 | 5.3 | 93.1 | 15.6 | 81.5 |
| Luna Experience 400 SC | BECF | 1.6 | 94.6 | 3.1 | 96.0 | 14.1 | 83.3 |
| Switch 62,5 WG | | | | | | | |

The results of both aforementioned experiments conducted under provocative conditions on apples and grapes (Table 10, Table 11), carried out under high agrotechnical standards and production intensity, clearly indicate the high effectiveness and thus the high suitability of both *Pantoea agglomerans* T16/8 strain strain for use in combating gray mold. The achieved effectiveness results of the strain were at least as good as the recognized standard, represented by the Serenade ASO preparation and the chemical protection program.

### Example 4

Application of *Pantoea agglomerans* T16/8 strain for plant protection against alternariosis caused by ***Alternaria*** species.

The assessment of the effectiveness of *Pantoea agglomerans* T16/8 strain in reducing alternariosis was conducted in field experiments on cabbage and carrots.

The first experiment was conducted on cabbage growing in an experimental field. The experiment was set up in a randomized block design with 4 replications and 2 rows of plants per rows (20 plants per plot). It included 6 combinations. For spraying, suspensions were used containing *Pantoea agglomerans* T16/8 strain at a concentration of 10⁷ colony-forming units per milliliter (CFU/ml). As a comparison, the experiment also included the standard biological product Serenade ASO and the standard chemical product Scorpion 325 SC. The suspensions were applied preventively - before the appearance of disease symptoms. Five spraying treatments were carried out on the cabbage plants. On the day of each spraying treatment, the degree of plant infection was determined using an 8-point scale: (0-0%; 1-1 %; 2-5%; 3-15%; 4-25%; 5-50%; 6-75%; 7-100%). The obtained research results were statistically analyzed using analysis of variance. To determine differences between means, Duncan's test was used at a significance level of α=0.05. The effectiveness of the preparations was evaluated using the modified Abbott's formula. Table 12 presents the assessment of the effectiveness of the used suspensions in reducing the development of alternariosis on cabbage.

**Table 12**

| Combination | % leaf infection/% efficacy | | | | | | | |
|---|---|---|---|---|---|---|---|---|
| | 1st assessment | | 2nd assessment | | 3rd assessment | | 4th assessment | |
| Control | 10.5 | - | 21.1 | - | 27.9 | - | 35.7 | - |
| T16/8 | 2.9 | 72.4 | 11.2 | 46.9 | 15.5 | 44.4 | 21.2 | 40.6 |
| Serenade ASO | 3.0 | 70.3 | 12.1 | 41.2 | 17.1 | 38.4 | 23.4 | 35.0 |
| Scorpion 325 SC | 0.0 | 100 | 0.5 | 97.6 | 1.0 | 96.4 | 1.5 | 95.8 |

Next, the effectiveness of *Pantoea agglomerans* T16/8 in limiting carrot alternariosis was evaluated. The experiment was conducted in field conditions on carrots grown in an experimental field. The experiment included 6 combinations and was set up in a randomized block design with 4 replications of 2 rows of plants each. For the sprays, aqueous suspensions containing either *Pantoea agglomerans* T16/8 strain were used at a concentration of 10⁷ CFU/ml. As a comparison, standard biological product Serenade ASO and chemical Scorpion 325 SC were used. The tested formulations were applied preventively, before the appearance of disease symptoms. Five spraying treatments were conducted on the carrot plants. On the day of each spraying treatment, the degree of plant infection was determined using an 8-step scale: (0-0%; 1-1%; 2-5%; 3-15%; 4-25%; 5-50%; 6-75%; 7-100%). The obtained research results were statistically analyzed using analysis of variance. To determine differences between means, the Duncan test was used with a significance level of α=0.05. The effectiveness of the treatments was assessed using the Abbott's corrected formula. Table 13 presents the assessment of the effectiveness of the tested formulations in limiting the development of carrot alternariosis.

**Table 13**

| Combination | % leaf infection/% efficacy | | | | | | | |
|---|---|---|---|---|---|---|---|---|
| | 3rd assessment | | 3rd assessment | | 3rd assessment | | 3rd assessment | |
| Control | 10.1 | - | 25.2 | - | 43.6 | - | 62.1 | - |
| T16/8 | 4.0 | 60.4 | 11.5 | 55.5 | 30.0 | 31.2 | 49.4 | 20.5 |
| Serenade ASO | 7.7 | 23.8 | 19.2 | 23.0 | 39.1 | 10.3 | 58.6 | 5.6 |
| Scorpion 325 SC | 0.0 | 100 | 0.5 | 98.0 | 1.1 | 97.5 | 3.9 | 93.7 |

The results of both experiments conducted on cabbage and carrots under provoking conditions (Table 12, Table 13) - with a high level of agronomy and production intensity - clearly indicate a high effectiveness and thus a high suitability of *Pantoea agglomerans* T16/8 strain for combating alternariosis. The achieved effectiveness results of the strain, according to the invention, were at least as good as or even better than the recognized standard, represented by Serenade ASO - a biological standard product.

### Example 5

Application of *Pantoea agglomerans* T16/8 strain for plant protection against anthracnose caused by the fungus *Glomerella acutata,* mainly known in the conidial stage as *Colletotrichum acutatum.*

The effectiveness of *Pantoea agglomerans* T16/8 in combating apple anthracnose during fruit storage was evaluated. The treatments were initiated during the flowering period and subsequently continued before harvest, 4, 3, 2, and 1 week before harvest, as well as "drenching" or immersing the apples in biopreparation solutions immediately after harvest. The evaluation was conducted after the fruit storage, comparing the number of apples with disease symptoms in the experimental combinations sprayed with a suspension containing *Pantoea agglomerans* T16/8 at a concentration of 10⁷ CFU/ml with the control combination (not sprayed). The first evaluation was conducted after more than 5 months of storage in a common cold room at a temperature of 2°C (1.8 - 2.2°C) and relative humidity of 90 - 92%, and the second evaluation was performed after 7 days of storage at a temperature of 15-20°C. In each combination, 1000 fruit samples were evaluated (4 repetitions x 250 samples per repetition), counting healthy and symptomatic fruit. The results were expressed as the percentage of affected fruit in relation to all evaluated fruit in the given combination. Statistical analysis was performed using the analysis of variance method, and differences between means were assessed using the Newman - Keuls test at a significance level of 5%. Symbols A, B, C, D, E, F, G - indicate the application timings (A - full bloom, B - petal fall, C, D, E, F - respectively 4, 3, 2, 1 week before harvest, G - "drenching" of fruit). For comparative purposes, commercial products Captan 80 WDG and Luna Experience 400 S.C. were also tested. The results are presented in table 14.

**Table 14**

| Combination | Treatment dates/concentration | 1st assessment after fruit storage in the cold room | |
|---|---|---|---|
| | | *Colletotrichum* spp. | Efficacy [%] |
| Control | - | 0.5 | - |
| Captan 80 WDG Luna Experience 400 SC | ABC 1.9 kg | 0.3 | 40 |
| | E 0.75 | | |
| T16/8 | CDEFG 10⁷ | 0 | 100 |

The results of the experiment conducted under provocative conditions - with a high level of agronomy and production intensity - clearly indicate a high effectiveness and consequently, a high suitability of the T16/8 strain for controlling anthracnose caused by *Colletotrichum spp.* The achieved efficacy results were at least as good as or better than established chemical standards (Captan 80 WDG, Luna Experience 400 SC).

### Example 6

Application of *Pantoea agglomerans* T16/8 strain for plant protection against **brown rot (moniliosis)** caused by fungi from the ***Monilinia spp.*** genus.

*In vitro* evaluation of the antagonistic properties of *Pantoea agglomerans* T16/8 against *Monilinia fructicola* was conducted. Initially, studies were carried out to assess the inhibition of *Monilinia fructicola* growth by *Pantoea agglomerans* T16/8 strain. The research was conducted on PDA medium (potato dextrose agar) as a co-culture of *Pantoea agglomerans* T16/8 strain and *Monilinia fructicola* fungus, observing the suppression of fungal growth. The zone of growth inhibition of the pathogenic *Monilinia fructicola* fungus by the T16/8 strain after 5 and 7 days is presented in Table 15. The numerical values presented in the table represent the radius from the colony's edge to the edge of the growth inhibition zone in millimeters.

**Table 15**

| Strain | Zone inhibition after 5 days [mm] | Zone inhibition after 7 days [mm] |
|---|---|---|
| Control | 0 | 0 |
| T16/8 | 15 | 5 |

Next, a study was conducted on 'Rajka' apple variety harvested at the mature collection stage (BBCH 87). A "drenching" method was employed, involving the immersion of fruits placed in crates with suspensions of the tested T16/8 strain, each separately. Apples used in the experiment during the pre-harvest period were not treated chemically. The "drenching" was performed a few hours after harvesting. The biological efficacy of the tested bacteria in controlling brown rot of pome fruit trees was evaluated after fruit storage, by comparing the number of apples with disease symptoms in the experimental combinations sprayed with suspensions of T16/8 strain and the control combination (unsprayed). Aqueous suspensions containing T16/8 strain were used at a concentration of 10⁷ CFU/ml. The first assessment was conducted after 3.5 months of apple storage in a common cold storage at a temperature of 2°C (1.8 - 2.2°C) and relative humidity of the air at 90 - 92%. The second and third assessments were carried out after 7 and 14 days of apple storage, respectively, at a temperature of 15-20°C. In each combination, 440 fruits were evaluated (4 repetitions x 110 fruits per repetition), counting healthy and affected apples. The results were expressed as the percentage of diseased fruits in relation to all evaluated apples in a given combination. The data were statistically analyzed using the analysis of variance method. Differences between means were assessed using the Newman-Keuls test at a significance level of 5%. The first assessment was performed after cold storage. The second assessment was conducted after storage at 15-20°C for 7 days. The third assessment was carried out after an additional 7 days of storage at 15-20°C. Table 16 presents the efficacy of the T16/8 strain used in the study during apple storage.

**Table 16**

| Combination | I assessment after fruit storage in the cold room | | II assessment after fruit storage at room temperature | | III assessment after fruit storage at room temperature | |
|---|---|---|---|---|---|---|
| | *Monilinia* spp. | Efficacy [%] | *Monilinia* spp. | Efficacy [%] | *Monilinia* spp. | Efficacy [%] |
| Control | 0.3 | - | 0.3 | - | 0.3 | - |
| T16/8 | 0 | 100 | 0 | 100 | 0 | 100 |

The results of the presented experiment, conducted under provocative conditions - with a high level of agronomic practices and production intensity, clearly indicate a high effectiveness and, consequently, a high suitability of the T16/8 strain for use in the control of apple brown rot caused by fungi of the genus *Monilinia spp.*

### Example 7

Application of *Pantoea agglomerans* T16/8 strain for plant protection against **bull's eye rot** caused by fungi of the ***Neofabrea* spp.**

*In vitro* assessment of the antagonistic properties of *Pantoea agglomerans* T16/8 bacterial strain against *Neofabrea* spp. fungi was conducted. Initially, studies were conducted to determine the growth inhibition of *Neofabrea spp.* by the strain *Pantoea agglomerans* T16/8. The experiments were conducted on PDA medium (potato dextrose agar), as co-cultures of *Pantoea agglomerans* T16/8 and *Neofabrea spp.,* observing the suppression of fungal growth. The experiments were conducted separately for each strain. The zone of growth inhibition of the pathogenic *Neofabrea spp.* fungi by the T16/8 strain after 14 days is presented in Table 17. The numerical values provided in the table indicate the radius from the edge of the colony to the edge of the growth inhibition zone in millimeters.

**Table 17**

| Strain | Zone of growth inhibition after 14 days [mm] |
|---|---|
| Control | 0 |
| T16/8 | 2 |

The inhibition of pathogen growth by the tested strain indicates the ability of T16/8 strain to produce substances with fungicidal or fungistatic properties.

Subsequently, the efficacy of *Pantoea agglomerans* T16/8 strain in controlling bull's eye rot in apples during fruit storage was evaluated. The initial treatments were administered during the flowering period, followed by treatments in the pre-harvest period 4, 3, 2, and 1 week before harvest, as well as "drenching" or immersing apples in suspensions of the tested strain T16/8, each separately, immediately after harvest. The biological efficacy of the tested bacterial strain in controlling bull's eye rot in apples was evaluated after fruit storage, based on a comparison of the number of apples with disease symptoms in the experimental combinations sprayed with aqueous suspensions of T16/8 strain and the control combination (unsprayed). Suspensions containing T16/8 strain were used at a concentration of 10⁷ CFU/ml. The first assessment was conducted after over 5 months of apple storage in a regular cold storage at a temperature of 2°C (1.8 - 2.2°C) and relative humidity of 90 - 92%, while the second assessment was conducted after 7 days of apple storage at a temperature of 15-20°C. In each combination, 1000 fruit samples were evaluated (4 repetitions x 250 samples per repetition), counting both healthy and affected apples. The results were expressed as the percentage of diseased fruit in relation to all evaluated apples in a given combination. Statistical analysis was performed using the analysis of variance method. Differences between means were assessed using the Newman-Keuls test at a significance level of 5%. Table 18 presents the efficacy of T16/8 strain in controlling bull's eye rot in apples during fruit storage. Symbols A, B, C, D, E, F, G - represent application timings (A - full blooming, B - petal fall, C, D, E, F - respectively 4, 3, 2, 1 week before harvest, G - "drenching" of fruit). Comparative studies were conducted for commercial products Captan 80 WDG and Luna Experience 400 S.C.

**Table 18**

| Combination | Application date/dose/concentration | Bull's eye rot caused by *Neofabraea* spp. | Efficacy [%] |
|---|---|---|---|
| Control | - | 1.4 | - |
| Captan 80 WDG Luna Experience 400 SC | ABC 1.9 kg | 0.3 | 78.6 |
| | E 0.75 | | |
| T16/8 | CDEFG 10⁷ | 0.4 | 71.4 |

The results of the presented experiment, conducted under provocative conditions - with a high level of agronomy and production intensity, clearly indicate a high effectiveness and therefore a high suitability of the tested T16/8 strain for controlling bull's eye rot in apples caused by fungi of the *Neofabrea spp.* genus. The achieved efficacy results of T16/8 strain were at least as good as, if not better than, established chemical standards (Captan 80 WDG, Luna Experience 400 SC).

### Example 8

Application of *Pantoea agglomerans* T16/8 strain in the reduction of **powdery mildew** on carrots caused by the fungus ***Erysiphe heraclei.***

The experiment was conducted in field conditions, on carrots grown in an experimental field. The experiment comprised 6 combinations and was set up in a randomized block design with 4 replications of 2 rows of plants. Aqueous suspensions were applied, containing *Pantoea agglomerans* T16/8 strain at a concentration of 10⁷ CFU/ml. Suspensions were applied preventively - before the appearance of disease symptoms. As a comparison, a standard biological product, Serenade ASO, was used in the experiment. Five plant sprayings were carried out. On the day of each spraying treatment, the degree of plant infection was determined using an 8-point scale: (0-0%; 1-1%; 2-5%; 3-15%; 4-25%; 5-50%; 6-75%; 7-100%). The obtained research results were statistically processed using an analysis of variance method. To determine differences between means, Duncan's test was used, with a significance level of α=0.05. The efficacy of the preparations was assessed using the modified Abbott formula. Table 19 presents the efficacy assessment of T16/8 strain in limiting the development of powdery mildew on carrots (*Erysiphe heraclei*).

**Table 19**

| Combination | % leaf infection/% efficacy | | | | | |
|---|---|---|---|---|---|---|
| | 1st assessment | | 2nd assessment | | 3rd assessment | |
| Control | 4.8 | - | 7.5 | - | 10.1 | - |
| T16/8 | 0.0 | 100 | 1.8 | 76.0 | 3.1 | 69.3 |
| Serenade ASO | 0.0 | 100 | 2.0 | 73.3 | 3.6 | 64.6 |

The results of the presented experiment, conducted under provocative conditions - with a high level of agronomy and production intensity, clearly indicate a high efficacy and thus high utility of the T16/8 strain in controlling powdery mildew on carrots caused by the fungus *Erysiphe heraclei.* The achieved efficacy results of the T16/8 strain were at least as good as or even better than the recognized product Serenade ASO.

### Example 9

Application of *Pantoea agglomerans* T16/8 strain for plant protection against fire blight caused by the bacterium *Erwinia amylovora.*

Research was conducted to assess the inhibition of *Erwinia amylovora* growth under *in vitro* conditions on the following substrates: NAS (2.3% nutrient agar with the addition of sucrose to a final concentration of 5%), King's B (as described by King, E.O., Ward, M.K., Raney, D.E. in "Two simple media for the demonstration of pyocyanin and fluorescin", J. Lab. Clin. Med. 1954, 44(2), 301-7), LB (tryptone 1%, NaCl 1%, yeast extract 0.5%, agar 2%), and NAG (nutrient agar 2.3%, glycerol 16 ml). The method described by Mikiciński (Mikiciński A, Sobiczewski P., Pulawska J., Maciorowski R. in "Control of fire blight (Erwinia amylovora) by a novel strain 49M of Pseudomonas graminis from the phyllosphere of apple (Malus spp.)", Eur J Plant Pathol, 2016, 145:265-276) was applied. *Pantoea agglomerans* T16/8 strain was spotinoculated onto appropriate media in the center of Petri dishes. After 3 days of incubation at 26°C, bacteria were killed using chloroform vapors, and the surface of the medium was covered with 4 ml of cooled soft agar containing 100 µl of Ea659 suspension (10⁷ CFU ml⁻¹). The inhibition zones of the pathogenic bacterium *Erwinia amylovora* by the T16/8 strain on various substrates after 24 and 48 hours of incubation at 26°C were measured. The tests were conducted in 6 repetitions. The inhibitory zone of *Erwinia amylovora* growth by the T16/8 strain on different substrates after 24 and 48 hours is presented in Table 20. The numerical values in the table indicate the radius from the edge of the colony to the edge of the growth inhibition zone in millimeters.

**Table 20**

| Strain | Medium | | | | | | | |
|---|---|---|---|---|---|---|---|---|
| | NAS | | LB | | King's B | | NAG | |
| | 24 h | 48 h | 24 h | 48 h | 24 h | 48 h | 24 h | 48 h |
| T16/8 | 7.5 | 7.5 | 0 | 0 | 0 | 0 | 4.3 | 0 |

Strain T16/8 exhibited a growth inhibition zone against *Erwinia amylovora* on two out of the four tested media.

Subsequently, another experiment was conducted under laboratory conditions on pear fruit blossoms. The blossoms of the "Conference" pear variety were sliced transversely into sections and immersed in a water suspension of strain. The suspension contained *Pantoea agglomerans* T16/8, at a concentration of 10⁷ CFU/ml, or sterile distilled water for the control. The experiment was conducted separately for each strain. The slices were placed on damp filter paper at the bottom of Petri dishes with a diameter of 20 cm. After 6 hours, infection was performed by spraying the surface of the slices with an inoculum of the pathogenic bacteria *Erwinia amylovora* (strain Ea 659) at a concentration of 10⁷ CFU/ml using a manual sprayer. Each combination consisted of 40 blossoms (4 repetitions). The effectiveness (S) of the T16/8 strain against fire blight on pear blossoms is presented in Table 21. Comparative tests were conducted for commercial products Switch 62.5 WG, Miedzian 50WP BlightBan A506, and BlightBan C9-1.

**Table 21**

| Combination | Assessment after 2 days | S [%] | Assessment after 5 days | S [%] | Assessment after 7 days | S [%] |
|---|---|---|---|---|---|---|
| Positive control | 0.93 | - | 3.0 | - | 4.0 | - |
| Switch 62,5 WG | 0.48 | 48.4 | 2.8 | 6.7 | 3.5 | 12.5 |
| 0,1% | | | | | | |
| BlightBan^{®} A506 | 0.0 | 100.0 | 0.15 | 95.0 | 0.63 | 84.2 |
| 10⁸ jtk/m | | | | | | |
| BlightBan^{®} C9-1 | 0.0 | 100.0 | 0.0 | 100.0 | 0.0 | 100.0 |
| 10⁸ jtk/m | | | | | | |
| T16/8 | 0.0 | 100.0 | 0.0 | 100.0 | 0.0 | 100.0 |
| 10⁸ CFU/ml | | | | | | |
| Miedzian 50WP | 0.0 | 100.0 | 0.23 | 92.3 | 0.43 | 89.2 |
| 0.15% | | | | | | |
| Miedzian 50WP | 0.0 | 100.0 | 0.0 | 100.0 | 0.0 | 100.0 |
| 0.3% | | | | | | |

The results of the presented laboratory experiment clearly indicate a high effectiveness and thus a high suitability of the T16/8 strain in combating fire blight caused by the bacterium *Erwinia amylovora.* The achieved effectiveness of the T16/8 strain was at least as good as, if not better than, recognized biological standards (BlightBan^{®} A506, BlightBan^{®} C9-1) and chemical standard (Miedzian 50WP).

### Example 10

Utilization of *Pantoea agglomerans* T16/8 strain for the protection of apples against **blue mold** caused by fungi of the genus *Penicillium **(Penicillium expansum),*** and for the protection against carrot root soft rot caused by bacteria ***Pectobacterium carotovorum.***

An *in vitro* assessment of the antagonistic properties of *Pantoea agglomerans* T16/8 strain against fungi of the genus *Penicillium spp.* was conducted. Firstly, the inhibition of growth in *Penicillium spp.* by the strain *Pantoea agglomerans* T16/8 was investigated. The study was performed on PDA medium (potato dextrose agar), with co-culturing of *Pantoea agglomerans* T16/8 strain along with *Penicillium spp.* The inhibition of pathogen growth was observed. With strain concentration in the suspension being 10⁷ CFU/ml. The growth inhibition zones of the *Penicillium spp.* pathogen by the T16/8 strain, observed after 5 and 7 days, were presented in Table 22.

**Table 22**

| Strain | *Penicillium expansum* growth inhibition zone after 5 days [mm] | *Penicillium expansum* growth inhibition zone after 7 days [mm] |
|---|---|---|
| Control | 0 | 0 |
| T16/8 | 20 | 0 |

The numerical values presented in the table determine the radius from the edge of the colony to the edge of the growth inhibition zone in mm. The inhibition of pathogen growth by the tested strain indicates the ability of T16/8 strain to produce substances with fungicidal or fungistatic properties.

The effectiveness of *Pantoea agglomerans* T16/8 bacterial strain in combating soft apple rot was examined. During the apple inoculation, two isolates of the pathogen *Penicillium expansum* (Isolate P8 and P11) were utilized, originating from diseased apples from different orchards. Spore suspensions of the fungus *Penicillium expansum* were obtained by washing 7-day-old cultures with distilled water. Then, 100 ml of the spore suspension was placed in a 250 ml Erlenmeyer flask and shaken to break up spore clusters. The concentration of conidial spores was adjusted to 10⁶ CFU/ml for isolate P8 and 10⁵ CFU/ml for P11. After about 2 hours of shaking, bacterial suspensions were standardized using a Bürker chamber. "Gala" variety apples were used in the study, which were stored in a regular refrigerator at 2°C immediately after harvesting. The apples were preliminarily cleaned by rinsing in water. After drying, the skin was disinfected using cotton soaked in 95% ethanol. Four wounds, 5 mm in diameter and 3 mm deep, were made symmetrically on the circumference in the central part of each fruit using a cork borer. Then, 30 µl of bacterial suspension was introduced into each wound. Two water suspensions were used, containing either *Pantoea agglomerans* T16/8 strain at a concentration of 10⁷ CFU/ml. The study was conducted in parallel and separately for each strain. After 1 hour, 30 µl of the corresponding isolate of *P. expansum* spore suspension (P8 or P11) was added. The spore concentration was adjusted to 10⁶ /ml. In the control combination, 30 µl of sterile distilled water was introduced into the wounds, and after 1 hour, 30 µl of the appropriate *P. exansum* isolate inoculum was added. The apples were stored for 5 days at room temperature (about 19°C) and relative humidity above 90%. The effectiveness of T16/8 strain against apple rot in the apple study is presented in Table 23.

**Table 23**

| Strain | Pathogen name and isolate number | | | |
|---|---|---|---|---|
| | *Penicillium expansum* P8 10⁶ | Efficacy [%] | *Penicillium expansum* P11 10⁵ | Efficacy [%] |
| Control | 28.8 | - | 18.2 | - |
| T16/8 10⁷ | 17.8 | 38 | 12.5 | 31 |

The results of the presented laboratory experiment clearly indicate the effectiveness and thus the usefulness of T16/8 strain in combating soft rot caused by the fungus *Penicillium expansum.*

In the next study, the effectiveness of *Pantoea agglomerans* T16/8 strain in limiting soft rot of carrot roots during storage, caused by the bacterium *Pectobacterium carotovorum,* was evaluated. Carrots growing in the experimental field were sprayed four times during vegetation, every 7 days (all plots), alternately with chemical preparations Amistar 250 SC, Zato 50 WG, Score 250 EC, and Scorpion 325 SC. Then, one month before harvesting, three treatments were performed every 10 days using suspensions containing T16/8 strain. A water suspension containing T16/8 strain was used at a concentration of 10⁷ CFU/ml. Subsequently, carrots (100 pieces from each combination) were placed in a cold room at a temperature of 4°C. The degree of root infection was assessed three times: on the day of removal from storage and after 10 and 20 days of root storage at room temperature. The degree of root infection was determined using an 8-point scale: (0-0%; 1-1 %; 2-5%; 3-15%; 4-25%; 5-50%; 6-75%; 7-100%). The results were statistically analyzed using an analysis of variance. Differences between means were assessed using the Duncan test at a significance level of 5%. The effectiveness of the tested agents was calculated using the Abbott formula. Comparative studies were also conducted for commercial preparations Serenade ASO and Scorpion 325 SC. The results are presented in Table 24.

**Table 24**

| Combinations | | Bacterial soft rot (*Pectobacterium carotovorum*) | | | | | |
|---|---|---|---|---|---|---|---|
| | | Average % of head infection | | | Efficacy [%] | | |
| | | Observation data | | | Observation data | | |
| | | after removal from the cold storage | after 10 days of storage at room temperature | after another 10 days | after removal from the cold storage | after 10 days of storage at room temperature | after another 10 days |
| 1 | Control | 0.6 | 2.5 | 6.0 | - | - | - |
| 2 | T16/8 | 2.22 | 5.21 | 8.9 | - | - | - |
| 3 | Serenade ASO | 0.36 | 6.5 | 12.6 | 40 | - | - |
| 4 | Scorpion 325 SC | 0.43 | 1.0 | 3.0 | 28.3 | 60 | 50 |

In the subsequent experiment, the effectiveness of the *Pantoea agglomerans* T16/8 strain was evaluated on the health of carrot roots during their post-harvest storage. The carrot tops were protected using chemical preparations with four treatments every 7 days. After harvesting, the carrot roots were soaked in a suspension containing the T16/8 strain at a concentration of 10⁷ CFU/ml. Subsequently, carrots (100 pieces from each combination) were placed in a cold room at a temperature of 4°C. The degree of root infection was assessed three times: on the day of removal from storage and after 10 and 20 days of root storage at room temperature. The degree of root infection was determined using an 8-point scale: (0-0%; 1-1 %; 2-5%; 3-15%; 4-25%; 5-50%; 6-75%; 7-100%). The results were statistically analyzed using an analysis of variance. Differences between means were assessed using the Duncan test at a significance level of 5%. The effectiveness of the tested agents was calculated using the Abbott formula. The results are presented in Table 25.

**Table 25**

| Combination | | Bacterial soft rot *- Pectobacterium carotovorum* | | | | | |
|---|---|---|---|---|---|---|---|
| | | Average % of root infection | | | Effectiveness [%] | | |
| | | Observation data | | | Observation data | | |
| | | On the day of exposure | After 10 days | After 20 days | On the day of exposure | After 10 days | After 20 days |
| 1 | Control | 7.75 | 12.16 | 18.08 | - | - | - |
| 2 | T16/8 | 0.49 | 3.49 | 8.91 | 93.7 | 71.3 | 50.7 |
| 3 | Serenade ASO | 11.0 | 25.33 | 32.24 | - | - | - |

The results of the above-mentioned experiments, conducted under provocative conditions with a high level of agrotechnology and production intensity, clearly indicate a high effectiveness and consequently high utility of the T16/8 strain for controlling root rots caused by *Pectobacterium carotovorum.* The achieved effectiveness results for the T16/8 strain were better than the recognized standard Serenade ASO.

## Claims

1. *Pantoea agglomerans* T16/8 strain, deposited in the Polish Collection of Microorganisms PCM at the Institute of Immunology and Experimental Therapy of the Polish Academy of Sciences, named after Ludwik Hirszfeld in Wroclaw, under number B/00272, on November 26, 2019.

2. The use of strain *Pantoea agglomerans* T16/8 deposited in the Polish Collection of Microorganisms PCM at the Institute of Immunology and Experimental Therapy of the Polish Academy of Sciences, named after Ludwik Hirszfeld in Wroclaw, on November 26, 2019, deposit number B/00272, as a means for plant protection against diseases caused by bacterial and/or fungal pathogens.

3. The use according to claim 2, **characterized in that** *Pantoea agglomerans* T16/8 strain is applied in the form of an aqueous suspension to the plant surface, through spraying, soaking the plants or its parts under storage conditions, under cover, and in field conditions, with the number of strain cells in the suspension being at least 10⁷ CFU/ml.

## Patentansprüche

1. *Pantoea agglomerans* Stamm T16/8, hinterlegt in der Polnischen Sammlung von Mikroorganismen PCM am Institut für Immunologie und experimentelle Therapie der Polnischen Akademie der Wissenschaften namens Ludwik Hirszfeld in Wroctaw, unter der Nummer B/00272, am 26. November 2019.

2. Verwendung des Stammes *Pantoea agglomerans* T16/8, hinterlegt in der Polnischen Sammlung von Mikroorganismen PCM am Institut für Immunologie und experimentelle Therapie der Polnischen Akademie der Wissenschaften namens Ludwik Hirszfeld in Wroctaw, am 26. November 2019, Hinterlegungsnummer B/00272, als Mittel zum Schutz von Pflanzen gegen Krankheiten, die durch bakterielle und/oder pilzliche Pathogene verursacht werden.

3. Verwendung nach Anspruch 2, **dadurch gekennzeichnet, dass** *Pantoea agglomerans* T16/8-Stämme in Form einer wässrigen Suspension auf die Pflanzenoberfläche aufgebracht werden, durch Besprühen, Einweichen der Pflanzen oder ihrer Teile unter Lagerbedingungen, unter Abdeckung und unter Feldbedingungen, wobei die Anzahl der Stammzellen in der Suspension mindestens 10⁷ KBE/ml beträgt.

## Revendications

1. Souche de *Pantoea agglomerans* T16/8, déposée le 26 novembre 2019 dans la Collection polonaise de micro-organismes PCM à l'Institut d'immunologie et de thérapie expérimentale de l'Académie polonaise des sciences Ludwik Hirszfeld à Wroctaw, sous le numéro B/00272.

2. Utilisation de la souche de *Pantoea agglomerans* T16/8, déposée le 26 novembre 2019 dans la Collection polonaise de micro-organismes PCM à l'Institut d'immunologie et de thérapie expérimentale de l'Académie polonaise des sciences Ludwik Hirszfeld à Wroctaw, sous le numéro B/00272, aux fins de protection des plantes contre les maladies causées par des agents pathogènes bactériens et/ou fongiques.

3. Utilisation selon la revendication 2, **caractérisée en ce que** les souches de *Pantoea agglomerans* T16/8 sont appliquées sous forme de suspension aqueuse à la surface des plantes par pulvérisation, imprégnant ainsi les plantes ou leurs parties dans des conditions de stockage, sous abri et en plein champ, le nombre de cellules de souche dans la suspension étant d'au moins 10⁷ UFC/ml.
